# EUROPEAN PATENT SPECIFICATION

(11) **EP 0 510 564 B1**
(45) Date of publication and mention of the grant of the patent: **10.04.1996**
(21) Application number: 92106753.4
(22) Date of filing: 21.04.1992
(51) Int. Cl.: C07H 15/04

(54) **Procedure for the preparation of surface-active agents derived from di- or tri-carboxylic acids**
Verfahren zur Herstellung von aus Di- oder Tri-Carboxylsäuren abgeleiteten Grenzflächenaktiven Mitteln
Procédé de préparation d'agents tensioactifs dérivés d'acides di- ou tri-carboxyliques

(30) Priority: 24.04.1991 IT MI911135
(43) Date of publication of application: 28.10.1992
(73) Proprietor: AUSCHEM S.p.A., I-20128 Milano (IT)
(72) Inventor: Bernardi, Paolo, I-20090 Vimodrone (MI) (IT); Fornara, Dario, I-28100 Novara (IT); Garlisi, Salvatore, I-13100 Vercelli (IT)
(74) Representative: Giambrocono, Alfonso, Dr. Ing.

(56) References cited:
- EP-A- 0 258 814
- WO-A-88/01640

## Description

The present invention relates to a process for the preparation of esters of di- or tri-carboxylic acids.

More specifically, the present invention relates to an esterification process of di- and tri-carboxylic acids with alkylpolyglucosides.

The esters of di- or tri-carboxylic acids with alkylpolyglucosides are known as surface-active agents, and are described, for example, in US Patents 4.797.481 and 4.806.275. These compounds are prepared by means of an esterification reaction of di- or tri-carboxylic acids with alkylpolyglucosides, carried out in mass or in an non-polar solvent such as hexane.

If the above reaction is carried out in bulk, there are draw-backs due to the unfavourable homogenizing of the reagents, which can cause undue local increases of the reaction temperature. This can give rise to phenomena such as partial degradation with caramelization of the glucose of the polyglucosidic chain and consequent undesired colouring of the product obtained.

For use in detergent compositions, where colouring of the surface-active agent is unacceptable, an onerous decolourizing treatment of the product is consequently necessary.

These coloured subproducts can also reduce the surface-active effect of the above mentioned esters.

If the reaction, on the other hand, is carried out in a solvent, it is necessary to separate said solvent from the reaction product.

It has now been found that these disadvantages of the processes already known in the art can be overcome by means of an improved process, whereby a better homogenizing of the reagents is obtained, thus minimizing the formation of coloured subproducts and greatly improving the yield into active product, and also avoiding the necessity of operating in the presence of a solvent.

The present invention consequently relates to a process for the preparation of esters of di- or tri-carboxylic acids having the general formula: 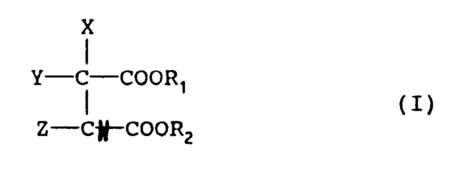
wherein:
X is H or the -CH₂COOR group;
Y and Z, the same or different, are H, -OH or together form a bond, on the condition that when X is -CH₂COOR, Y is -OH and Z is H;
R, R₁ and R₂, the same or different, represent a hydrogen atom or an A radical derived from a monoalkyl(C₆-C₁₆) glucose or polyglucose ether, on the condition that at least one of the above R, R₁ and R₂ is an A radical;
which consists of esterifying an acid having the general formula: 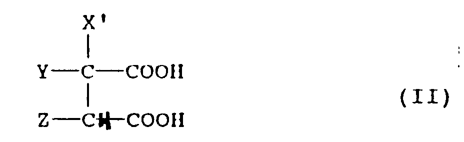
or the respective anhydride, or mixtures of these, with a compound having the formula:

A-OH (III)

wherein X' and H or the -CH₂COOH group, and Y, Z, A have the above-mentioned meaning, in the presence of a fatty alcohol containing from 6 to 16 carbon atoms in quantities of 15 to 50% by weight with respect to compound (II), by heating to a temperature of 120 to 140°C.

The water formed by the reaction is preferably distilled in continuous.

The esters obtained from the process of the present invention may be submitted to a final salification treatment with bases of alkaline or earth-alkaline metals, ammonia or amines.

Di- or tri-carboxylic acids included in the general formula (II) are citric, tartaric, malic, succinic and maleic acids.

Suitable glucose or polyglucose alkylethers having formula (III), which are well-known compounds and can be prepared with the usual methods and are sometimes available on the market, are, for example, those corresponding to the general formula: 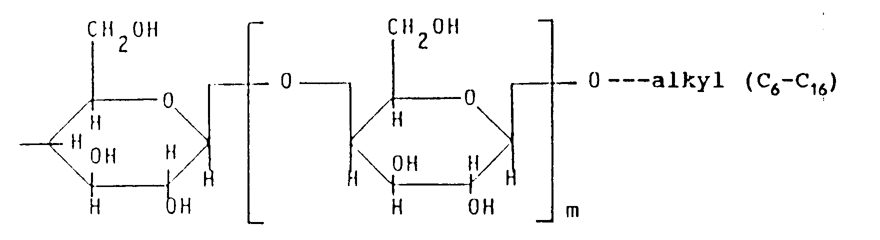
wherein m is an integer between 0 and 5.

Fatty alcohols containing from 6 to 16 carbon atoms suitable for the process of the present invention are, for example, octyl, decyl, dodecyl and tetradecyl alcohols or their mixtures.

The final salification treatment, can be carried out, for example, with sodium, potassium, magnesium, ammonium hydroxide, triethanolamine or monoethanolamine.

The process conditions of the present invention concern the esterification of di- or tri-carboxylic acids with polyglucose alkylethers and fatty alcohols.

The reaction product is therefore composed of esters of alkylpolyglucosides and esters of fatty alcohols which form part of the mixture of reagents.

The esters of alkylpolyglucosides can be separated from the esters of fatty alcohols using the known methods.

As the esters of fatty alcohols, however, have good surface-active properties and are perfectly compatible with the esters of alkylpolyglucosides, the mixtures of esters obtained from the process of the present invention may be suitably used as such for the formulation of detergent compositions.

Depending upon the reaction conditions used, and especially on the molar ratios between the acid and alkylpolyglucoside and between the acid and fatty alcohol, it is possible to prepare preferentially mono-, di- and, in the case of tri-carboxylic acids, tri-esters.

Examples of equations which represent the esterification reaction of an acid having formula (II) with a compound having formula (III) are contained in US Patent No. 4.797.481.

In the process of the present invention, it is particularly advantageous to use as a reagent a fatty alcohol whose alkylic chain is equal to the alkyl of radical A.

In this case the mixtures of products can be used directly and are obtained by the reaction of fatty alcohols with glucose.

In fact, these mixtures are composed of alkylpolyglucosides and the relative unreacted alcohols; the latter may even be present in considerable quantities (up to 50% and more).

The alkylpolyglucosides thus prepared, in order to be used as surface-active agents, had to be separated from the unreacted fatty alcohols. This separation treatment required the use of complicated and costly techniques, such as thin film evaporation.

By submitting the above mixtures to esterification, which constitutes a further aspect of the present invention, it is no longer necessary to separate the unreacted fatty alcohol and complete transformation of said mixtures into mixtures of surfact-active products is obtained.

In fact, both the esters of polyalkylglucosides and those of fatty alcohols, as also their salts and their mixtures, are extremely effective surface-active agents, also producing, if used in very limited percentages, a considerable decrease in surface stress, making it possible for them to be used as builders, dissolving agents and detergents in general.

As well as their excellent detergent properties, they have no toxic or irritating effect on the skin and eyes and have no acute toxicity if swallowed orally. In addition, they are highly biodegradable.

The esters of the present invention have proved to be compatible with most of the known surface-active agents and can therefore be formulated with these.

The esters of the present invention, owing to their combination of characteristics, have proved to be extremely flexible in various applications of surface-active agents.

Owing to their high detergent capacity together with the lack of toxic effects on the skin, hair, eyes, they are particularly suitable for cosmetic applications such as the preparation of liquid or cream skin detergents, shampoos and bath foams.

The following examples provide a better illustration of the present invention but do not limit it in any way.

### EXAMPLE 1

Preparation of an ester of citric acid with a monoalkylpolyglucoside.

### Esterification

162.1 g of citric acid and 300 g of a polyglucose with a polymerization degree of 2.6 etherified with a mixture of decyl alcohol and dodecyl alcohol, containing 29% by weight of the above mixture of alcohols in free form, are loaded, under a nitrogen flow, into a reactor equipped with a heating system, stirrer, thermometer, reagent inlet system and connected to a cooler equipped with a container to collect the reaction water.

The temperature is brought to 130°C under stirring and nitrogen flow, and the reaction mixture is kept at this temperature until the acidity number reaches a value of 211, 9±3.

The mixture is cooled to a temperature of 110-115°C, diluted with 455 g of water, cooled to 50°C and is finally discharged from the reactor.

910 g of a turbid liquid product are obtained, containing 50% of water, having an acidity value of 105.2 and saponification value of 157.4.

### Salification

200 g of the citric ester previously prepared and 109.3 g of demineralized water are loaded into a container equipped with stirrer, thermometer, drip funnel and water cooling system.

The mixture is stirred until a homogeneous mass is obtained and then 53.5 g of a 30% aqueous solution of NaOH is slowly added, under stirring, over a period of an hour, by means of the drip funnel, the temperature being kept at values of below 30°C.

362.8 g of a cloudy liquid product containing 30% by weight of citric ester salified with sodium are obtained.

A yellow vitreous solid is obtained from the solution, by removing the water by evaporation at 50°C for 16 hours under vacuum, mainly composed of the sodium salt of citric ester, having an acidity value of 3, a saponification number of 104.8, an ester value of 101.8 and a pH at 1% of 7.1.

### EXAMPLE 2

Preparation of an ester of maleic acid with a monoalkylpolyglucoside.

### Esterification

170.3 g of maleic anhydride and 470 g of a polyglucose with a polymerization degree of 2.6 etherified with a mixture of decyl alcohol and dodecyl alcohol, containing 29% by weight of the above mixture of alcohols in free form, are loaded, under a nitrogen flow, into a reactor equipped with a heating system, stirrer, thermometer and reagent inlet system.

The temperature is brought to 120°C under stirring and nitrogen flow, and the reaction mixture is kept at this temperature until the acidity number reaches the value of 152±3.

The mixture is then cooled to a temperature of 110-115°C, diluted with 640 g of water, cooled to 50°C and is finally discharged from the reactor.

1280 g of a turbid liquid product are obtained, containing 50% of water, having an acidity value of 75.7 and a saponification number of 151.1.

### Salification

250 g of maleic ester previously prepared and 144.3 g of demineralized water are loaded into a container equipped with a stirrer, thermometer, drip funnel and water cooling system.

The mixture is stirred until a homogeneous mass is obtained and 49.7 g of a 30% aqueous solution of NaOH, are then slowly added, under stirring, over a period of an hour, by means of the drip funnel, the temperature being kept at values lower than 30°C.

444.0 g of a cloudy liquid product containing 30% by weight of maleic ester salified with sodium, are obtained.

A yellow vitreous solid is obtained from the solution, by removing the water by evaporation at 50°C for 16 hours under vacuum, mainly composed of the sodium salt of maleic ester, having an acidity value of 2.4, a saponification number of 152.0, an ester number of 149.6 and a pH at 1% of 6.9.

### APPLICATIVE TESTS

The following tests were carried out on the products obtained in Examples 1 and 2, on the alkylpolyglucoside mixed with its alcohols used in Examples 1 and 2 (Example A is comparative) and on an alkylpolyglucoside with C₁₂-C₁₄ alcohols normally available on the market (Example B is comparative):

### - Surface tension

The surface tension of solutions having 0.1% of active substance was measured at 20°C using the du-Nouy method. The results, measured as dine/cm at a concentration of 1 g/l, are shown in the Table.

### - Foaming power

The capacity of producing foam was evaluated at 25°C in hard water 30°F using the Ross-Miles method. Measurements were carried out at concentrations of 0.5 g/l after 0 minutes (I) and after 5 minutes (II), and at concentrations of 1 g/l after 0 minutes (III) and after 5 minutes (IV). The results are shown in the Table.

**TABLE**

| Example | Surface tension | Foaming power | | | |
|---|---|---|---|---|---|
| | | I | II | III | IV |
| 1 | 27.9 | 95 | 90 | 110 | 100 |
| 2 | 28.0 | 120 | 115 | 150 | 145 |
| A | 28.5 | 30 | 25 | 45 | 40 |
| B | 28.7 | 30 | 25 | 35 | 30 |

## Claims

1. Process for the preparation of esters of di- or tri-carboxylic acids having the general formula: 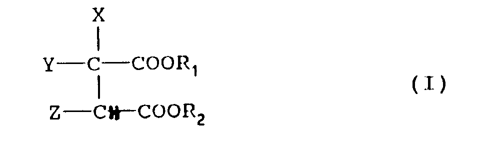 wherein:
X is H or the -CH₂COOR group;
Y and Z, the same or different, are H, -OH or together form a bond, on the condition that when X is -CH₂COOR, Y is -OH and Z is H;
R, R₁ and R₂, the same or different, represent a hydrogen atom or an A radical derived from a monoalkyl (C₆-C₁₆) glucose or polyglucose ether containing from 2 to 6 monomeric units of glucose, on the condition that at least one of said R, R₁ and R₂ is an A radical;
which consists of esterifying an acid having the general formula: 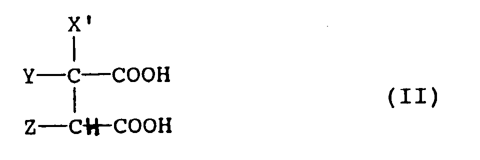 or the respective anhydride, or mixtures of these, with a compound having the formula:
A-OH (III)
wherein X' is H or -CH₂COOH group, and Y, Z, A have the meaning specified above, in the presence of a fatty alcohol containing from 6 to 16 carbon atoms in quantities of 15 to 50% by weight with respect to the compound (III), by heating to a temperature of 120 to 140°C.

2. Process in accordance with Claim 1, wherein the alkylic chain of the fatty alcohol is equal to that of the alkyl (C₆-C₁₆) of radical A.

3. Process in accordance with one or more of the previous Claims, wherein the product obtained is salified with bases of alkaline or earth-alkaline metals, ammonia or amines.

## Patentansprüche

1. Verfahren zur Herstellung der Esteren von Bi oder-Tricarbonsauren mit der allgemeinen Formel 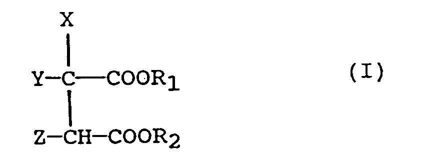 in der:
X, H oder - CH₂COOR- Gruppe ist
Y und Z, die gleich oder verschieden sein konnen, H oder-OH sind oder eine Verbindung herstellen, unter der Bedingung dass wenn X -CO₂COOR, Y - OH und Z H ist;
R,R₁ und R₂_{,} die gleich oder verschieden sein konnen, ein Wasserstoffatom oder einen A-Rest, der von einer Glukose oder Polyglukose (C₆- C₁₆) monoalkylethern stammt mit 2 bis 6 monomeren Glukoseneinheiten darstellen, unter der Bedingung dass mindestens eine der Gruppen R, R₁, R₂ ein A- Rest ist, wobei das Verfahren darauf beruht, dass man ein Sauer der allgemein Formel 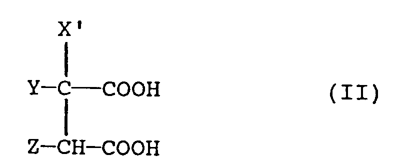 Anhydrid derselben oder Gemische desselben mit einer Verbindung der Formel
A―OH (III)
verethert
in der X' H oder eine -CH₂COOH Gruppe ist, und Y,Z,A bedeuten wie oben definiert ist,in der Gegenwart eines fetten Alkohols mit 6 bis 16 Wassestoffatom so viel als 15 und 50% G ewicht bezüglich der Verbindung (III), durch Erhitzen auf eine Temperatur von 120 bis 140°C.

2. Verfahren nach Anspruch 1, worin die Alkylkette des fetten Alkohols gleich wie jene des A- Rest Alkyls ist.

3. Verfahren nach einem der Anspruche 1 bis 2, worin das hergestellte Erzeugnis mit Basen von Alcali oder Erdalkalimetallen, Ammoniak oder Ammine in Salz verwandelt wird.

## Revendications

1. Procédé pour la préparation des esters de acides bi-ou tricarboxyliques ayant la formule générale 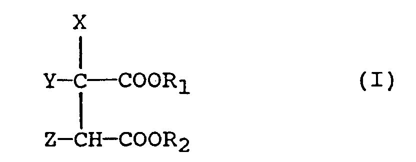 où
X est H ou le groupe -CH₂ COOR;
Y et Z, égals ou différents entre eux, sont H, -OH ou ensemble forment une liaison, à condition que quand X est -CH₂COOR, Y est - OH et Z est H;
R,R₁ et R₂., égals ou différents entre eux, représentent un atome de hydrogéne ou un radical A dérivé d'un monoalchil ( C₆-C₁₆ ) ether du glucose ou du polyglucose ayant de 2 à 6 unités monomèriques de glucose, à condition que au moins un des susdits R,R₁ et R₂ soit un radical A;
consistant à estérifier un acid de formule générale 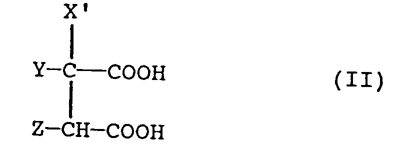 ou bien la respective anhydride ou mélanges de eux memes, avec un composé de formule
A―OH (III)
où X' est H ou le groupe -CH₂COOH et Y,Z,A ont le signifié indiqué ci-dessus, en présence d'un alcool gras, contenant de 6 à 16 atoms de carbone en quantitès comprises entre le 15 et le 50% en poids par rapport au composé (III), au moyen de chauffage à température comprise entre 120 et 140°C.

2. Procédé selon la revendication 1, dans lequel la chaine alcoylique de l'alcool gras est égale à quelle de l'alcoyle ( C₆-C₁₆) du radical A.

3. Procédé selon une des revendications précédentes , dans lequel le produit obtenu est ultérieurement salifié avec bases de métaux alcalins ou acalino terreux, ammoniac ou amines.
